# EUROPEAN PATENT APPLICATION

(11) **EP 4 742 263 A1**
(43) Date of publication of application: **13.05.2026**
(21) Application number: 25213393.9
(22) Date of filing: 04.11.2025
(51) Int. Cl.: G16H 20/40, A61N 5/10, G06N 20/00, G16H 50/70

(54) **CUSTOMIZED MACHINE-LEARNING TRAINING FOR RADIOTHERAPY CLINICS**

(30) Priority: 07.11.2024 US 202418940754
(71) Applicant: Siemens Healthineers International AG, 6312 Steinhausen (CH)
(72) Inventor: HAKALA, Mikko, 05200 Rajamäki (FI); CZEIZLER, Elena, 00390 Helsinki (FI); LAAKSONEN, Hannu, 02130 Espoo (FI)
(74) Representative: Mathisen & Macara LLP

(57) **Abstract**

Disclosed herein are methods (200) for selecting and preparing patient data to facilitate the adoption and customized training of machine-learning models in clinical settings, particularly for radiation therapy treatment planning. The disclosed embodiments streamline the customized training process through an automated workflow that includes prefiltering (320) patient metadata, retrieving (322) relevant DICOM files, optional data anonymization (326), and generation of training data. The data is then organized into a format suitable for machine-learning training. The embodiments discussed herein reduce manual labor, minimize errors, and accelerate the integration of machine-learning into clinical workflows, enabling clinics to train and implement predictive models that replicate specific clinical practices, thereby enhancing treatment precision and improving patient outcomes.

## Description

### TECHNICAL FIELD

This application relates generally to clinic-specific radiotherapy planning systems, and in particular, to customization of training machine-learning models for radiotherapy planning, to increase their operational efficiency.

### BACKGROUND

Radiation therapy treatment planning (RTTP) is a complex process that contains specific guidelines, protocols, and instructions adopted by different medical professionals, such as clinicians, medical device manufacturers, treating physicians, and the like. Due to the extreme nature of radiation emitted from radiotherapy machines, it is imperative that all the instructions are precisely followed. Field geometry, as used in the context of RTTP, refers to various attributes or settings of a radiotherapy machine while a patient receives a prescribed radiotherapy dose. For instance, a prescribing physician may identify a structure (e.g., the patient's organ to be treated or tumor to be eradicated) and a corresponding dosage. Moreover, other parties (e.g., clinicians or machine manufacturers) may determine positioning attributes (e.g., angles) of the gantry and the patient on the couch to provide optimum treatment.

In order to increase efficiency in this process, many clinics use their own machine-learning models that are trained using a central entity. For instance, an entity may provide a machine learning model to a particular clinic where the clinic can customize or fine-tune the machine learning model using their own patient data and/or clinic-specific rules and protocols. This customized machine-learning approach can be integrated into plan optimizer platforms wherein a machine-learning model trained using a cohort of patients is fine-tuned for a particular clinic. These approaches primarily aim to assist medical professionals and dosimetrists in creating high-quality, consistent treatment plans for cancer patients by leveraging past treatment data using their customized model for a particular clinic. Using this paradigm, each clinic can train its model (or adapt by further training a pre-trained model) using its own historical treatment data or any customized set of data. This allows the model to reflect that clinic's specific clinical practices and preferences, ensuring that the generated treatment plans align with their standards of the clinic.

However, clinic-specific radiotherapy planning approaches have faced some challenges. For instance, this system is labor-intensive and error-prone because it involves each clinic preparing data for its model training. For instance, clinicians must manually identify and select relevant patient data from a large and complex database to train a clinic-specific model. This process involves extracting treatment plans, ensuring the data is properly labeled, and organizing it into a format that can be used to train the model while ensuring data quality. This manual effort is time-consuming and requires a high level of expertise, making it a significant challenge for medical professionals. Moreover, the quality of the model will directly depend upon the subjective expertise of the medical professionals of each clinic, which is undesirable and inconsistent.

Finally, the models may not operate efficiently because they are sensitive to the variability in the data used for training. For instance, some models require homogeneous datasets to function effectively, meaning that any inconsistencies or outliers in the data can negatively impact the model's performance. This further complicates the data preparation process, as it necessitates careful selection and curation of data to ensure that it meets the necessary criteria for training.

### SUMMARY

According to a first aspect of the invention, there is provided a method for training and integrating a machine learning model for radiation therapy treatment planning, as defined in claim 1.

According to a second aspect of the invention, there is provided a computer-readable medium for training and integrating a machine learning model for radiation therapy treatment planning, as defined in claim 7.

According to a third aspect of the invention, there is provided a computer system for training and integrating a machine learning model for radiation therapy treatment planning, as defined in claim 13.

Optional features are defined in the dependent claims.

For the aforementioned reasons, there is a desire for a system that can adapt or otherwise customize a computer model (e.g., an AI or machine-learning or a more conventional model) for a particular clinic. Using the methods and systems discussed herein may allow for faster and more efficient training time, and sometimes, using fewer computing resources. Moreover, machine learning models that are trained using the methods and system discussed herein may be customized to clinics, such that their predictions are more accurate. Therefore, the methods and systems discussed herein provide functional and technical improvements specific to the field of machine learning.

The methods and systems discussed herein address the challenges associated with manual data handling in the training of machine-learning models for radiotherapy treatment planning. Currently, clinics must manually curate patient data from complex databases, a process that is both time-consuming and error-prone. This manual approach leads to inconsistencies and inefficiencies, as clinics need to extract relevant data, organize it, and ensure its quality for machine-learning model training. The methods and systems discussed herein provide an automated workflow that simplifies patient data selection, file mapping, anonymization, and final data preprocessing, making the process more efficient and reducing the reliance on manual effort.

The methods and systems discussed herein streamline data preparation for machine-learning model training so that operational efficiencies are achieved. This workflow begins by prefiltering patient data based on specific criteria from the clinic's database, identifying relevant cases and treatment types. The selected data can then be mapped to corresponding DICOM files (and all other relevant files) and metadata to ensure only necessary files are processed. An optional anonymization step may also be utilized if the data needs to be shared with external parties, safeguarding patient confidentiality. The data may then be transferred to a computational platform for final preprocessing, where it is organized into a format suitable for machine-learning model training, such as separating different treatment phases or filtering out specific cases like retreatments. Additionally, the methods and systems discussed herein can provide clustering data into different classes or data subsets to ensure consistency and minimize outliers, which enhances the effectiveness of the machine-learning model, its training, and its operation. By automating these steps, the methods and systems discussed herein reduce manual effort, minimize errors, and optimize data preparation for clinic-specific machine-learning model training, ultimately improving the quality and consistency of radiation therapy planning.

In some aspects, the techniques described herein relate to a method for training and integrating a machine learning (ML) model for radiation therapy treatment planning, the method including: receiving, by at least one processor, one or more radiotherapy treatment attribute for training the ML model; retrieving, by the at least one processor querying a database accessible to a clinic, the database storing treatment data associated with a set of previously treated patients, patient data corresponding to a subset of the set of the patients satisfying the one or more radiotherapy treatment attribute; concatenating, by the at least one processor, the retrieved patient data with one or more digital imaging and communications in medicine files or other medical files and corresponding metadata associated with a previously performed treatment of each patient within the subset of the set of patients; generating, by the at least one processor, a training dataset based on the patient data and the concatenated one or more digital imaging and communications in medicine files or other medical files and metadata by changing at least a file structure of the training dataset in accordance with a configuration file of the ML model; and training, by the at least one processor, the ML model using the training dataset, such that the ML model is customized to the clinic.

In some aspects, the techniques described herein relate to a method, wherein the machine learning model is only trained using the training dataset.

In some aspects, the techniques described herein relate to a method, wherein the machine learning model was previously trained using a secondary training dataset and is fine-tuned for the clinic.

In some aspects, the techniques described herein relate to a method, further including: anonymizing, by the at least one processor, at least one of the patient data, the concatenated digital imaging and communications in medicine files or other medical files, or the metadata.

In some aspects, the techniques described herein relate to a method, further including: clustering, by the at least one processor, the training dataset into a plurality of different classes of data.

In some aspects, the techniques described herein relate to a method, further including: when an outlier data point is identified, removing, by the at least one processor, the outlier data point within the training dataset.

In some aspects, the techniques described herein relate to a method, wherein the radiotherapy treatment attribute corresponds to a specific treatment technique.

In some aspects, the techniques described herein relate to a method, further including: de-duplicating, by the at least one processor, the training dataset by removing data associated with patients that satisfy a similarity threshold.

In some aspects, the techniques described herein relate to a computer-readable medium for training and integrating a machine learning (ML) model for radiation therapy treatment planning, the computer-readable medium including instructions that when executed cause a processor to: receive one or more radiotherapy treatment attribute for training the ML model; retrieve by the at least one processor querying a database accessible to a clinic, the database storing treatment data associated with a set of previously treated patients, patient data corresponding to a subset of the set of the patients satisfying the one or more radiotherapy treatment attribute; concatenate the retrieved patient data with one or more digital imaging and communications in medicine files or other medical files and corresponding metadata associated with a previously performed treatment of each patient within the subset of the set of patients; generate a training dataset based on the patient data and the concatenated one or more digital imaging and communications in medicine files or other medical files and metadata by changing at least a file structure of the training dataset in accordance with a configuration file of the ML model; and train the ML model using the training dataset, such that the ML model is customized to the clinic.

In some aspects, the techniques described herein relate to a computer-readable medium, wherein the machine learning model is only trained using the training dataset.

In some aspects, the techniques described herein relate to a computer-readable medium, wherein the machine learning model was previously trained using a secondary training dataset and is fine-tuned for the clinic.

In some aspects, the techniques described herein relate to a computer-readable medium, wherein the instructions further cause the processor to anonymize at least one of the patient data, the concatenated digital imaging and communications in medicine files or other medical files, or the metadata.

In some aspects, the techniques described herein relate to a computer-readable medium, wherein the instructions further cause the processor to cluster the training dataset into a plurality of homogeneous subsets.

In some aspects, the techniques described herein relate to a computer-readable medium, wherein the instructions further cause the processor to, when an outlier data point is identified, remove the outlier data point within the training dataset.

In some aspects, the techniques described herein relate to a computer-readable medium, wherein the radiotherapy treatment attribute corresponds to a specific treatment technique.

In some aspects, the techniques described herein relate to a computer-readable medium, wherein the instructions further cause the processor to de-duplicate the training dataset by removing data associated with patients that satisfy a similarity threshold.

In some aspects, the techniques described herein relate to a computer system for training and integrating a ML model for radiation therapy treatment planning, the computer system including a processor configured to: receive one or more radiotherapy treatment attribute for training the ML model; retrieve by the at least one processor querying a database accessible to a clinic, the database storing treatment data associated with a set of previously treated patients, patient data corresponding to a subset of the set of the patients satisfying the one or more radiotherapy treatment attribute; concatenate the retrieved patient data with one or more digital imaging and communications in medicine files or other medical files and corresponding metadata associated with a previously performed treatment of each patient within the subset of the set of patients; generate a training dataset based on the patient data and the concatenated one or more digital imaging and communications in medicine files or other medical files and metadata by changing at least a file structure of the training dataset in accordance with a configuration file of the ML model; and train the ML model using the training dataset, such that the ML model is customized to the clinic.

In some aspects, the techniques described herein relate to a computer system, wherein the machine learning model is only trained using the training dataset.

In some aspects, the techniques described herein relate to a computer system, wherein the machine learning model was previously trained using a secondary training dataset and is fine-tuned for the clinic.

In some aspects, the techniques described herein relate to a computer system, wherein the processor is further configured to anonymize at least one of the patient data, the concatenated digital imaging and communications in medicine files or other medical files, or the metadata.

In some aspects, the techniques described herein may relate to a computer system wherein the processor is further configured to cluster the training dataset into a plurality of consistent subsets.

In some aspects, the techniques described herein may relate to a computer system wherein the processor is further configured to, when an outlier data point is identified, remove the outlier data point within the training dataset.

In some aspects, the techniques described herein may relate to a computer system wherein the radiotherapy treatment attribute corresponds to a specific treatment technique.

In some aspects, the techniques described herein may relate to a computer system wherein the processor is further configured to de-duplicate the training dataset by removing data associated with patients that satisfy a similarity threshold.

### Examples

The retrieving step may be performed by the at least one processor querying the database accessible to the clinic. The database may store treatment data associated with the set of previously treated patients. The retrieving step may comprise retrieving patient data corresponding to a subset of the set of the patients satisfying the one or more radiotherapy treatment attribute(s).

Optionally, a patient satisfying a radiotherapy treatment attribute may comprise a patient having undergone radiotherapy having the attribute.

Optionally, the machine learning model is trained using only the training dataset.

Optionally, the machine learning model was previously trained using a secondary training dataset and is subsequently fine-tuned for the clinic.

### BRIEF DESCRIPTION OF THE DRAWINGS

Non-limiting embodiments of the present disclosure are described by way of example with reference to the accompanying figures, which are schematic and are not intended to be drawn to scale. Unless indicated as representing the background art, the figures represent aspects of the disclosure.
**FIG. 1** illustrates components of a clinic-specific radiotherapy planning system, according to an embodiment.
**FIG. 2** illustrates an example flow diagram of a process executed in a customized radiotherapy planning system, according to an embodiment.
**FIG. 3** illustrates an example flow diagram of a process executed in a customized radiotherapy planning system, according to an embodiment.

### DETAILED DESCRIPTION

Reference will now be made to the illustrative embodiments depicted in the drawings, and specific language will be used here to describe the same. It will nevertheless be understood that no limitation of the scope of the claims or this disclosure is thereby intended. Alterations and further modifications of the inventive features illustrated herein, and additional applications of the principles of the subject matter illustrated herein, which would occur to one skilled in the relevant art and having possession of this disclosure, are to be considered within the scope of the subject matter disclosed herein. Other embodiments may be used and/or other changes may be made without departing from the scope of the present disclosure. The illustrative embodiments described in the detailed description are not meant to be limiting of the subject matter presented.

In current clinical practice, radiation treatment plans are typically generated using general guidelines outlined by clinical protocols. These protocols provide the main criteria for plan acceptance, such as dose thresholds that aim to achieve favorable treatment outcomes while avoiding undesirable side effects. However, these protocols are often not exhaustive, offering only broad guidelines and leaving out specific criteria that planners frequently consider when aiming to generate high-quality treatment plans.

For example, while clinical protocols usually omit information on dose gradients around targets, planners often take this into account to ensure adequate sparing of organs at risk (OARs), especially those near the target areas. This lack of detail in protocols contributes to significant variability in treatment planning practices across different clinics, including preferences for trade-offs between target homogeneity and OAR sparing, prescription levels, and the choice of field geometry. Accordingly, when adopting AI/ML-based solutions to aid in plan generation, the machine-learning model may need to reflect the specific clinical practices of the hospital. This can be achieved by training or fine-tuning machine-learning models on the hospital's own data.

Using the methods and systems discussed herein, clinics can train their own models to predict dose-volume histograms (DVH) based on their historical treatment data. Similarly, other AI/ML-based models used for tasks like 3D dose prediction or field geometry setting must replicate clinic-specific practices. However, the challenge lies in the manual identification, selection, and processing of patient data required for training these models-a task that is both time-consuming and prone to errors.

Handling a clinic's patient data for machine-learning model training or radiation therapy treatment analysis involves several technical challenges. First, clinicians must extract relevant patient metadata from structured databases, such as treatment plans, structure sets, CT sets, and dose information stored in DICOM files or other medical files. This data must then be curated so that it remains usable for model development without requiring extensive filtering later in the process. Anonymization may be necessary if external parties are involved, adding another layer of complexity. Moreover, raw data may not be suitable for data scientists to use directly because it sometimes requires a series of preprocessing steps. These steps include prefiltering patient metadata, matching DICOM files, and other types of medical files, to the corresponding metadata, anonymizing the data if needed, transferring it to an analysis platform, and organizing the data into a format required for model training, such as arranging files in a structured folder or tabulating metadata. Only after these preprocessing steps can data scientists begin their actual work on model development or data analysis.

To address these technical challenges, the methods and systems discussed herein provide an automated workflow that streamlines patient data selection and preparation, facilitating the easier adoption and training of machine-learning models in clinics. By automating these tasks, the methods and systems discussed herein provide a more efficient, error-free process for handling clinic-specific patient data, enabling smoother integration of AI tools into clinical workflows.

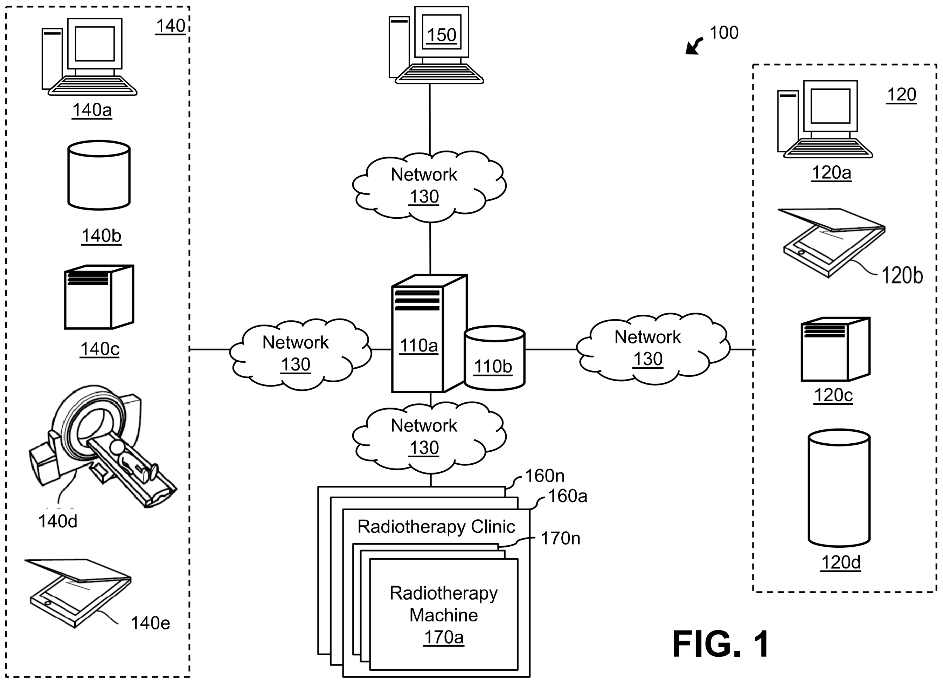

**FIG. 1** illustrates components of a clinic-specific radiotherapy planning system 100. The system **100** may include an analytics server **110a,** system database **110b,** electronic data sources **120a-d** (collectively electronic data sources **120),** end-user devices **140a-e** (collectively end-user devices **140),** an administrator computing device **150,** and radiotherapy clinics **160a-n** (collectively radiotherapy clinics **160).** The radiotherapy clinics **160** may be clinics at which patients may receive radiotherapy treatment, in some cases via one or more radiotherapy machines located within the clinic. The above-mentioned components may be connected to each other through a network **130.** Examples of the network **130** may include, but are not limited to, private or public LAN, WLAN, MAN, WAN, and the Internet. The network **130** may include wired and/or wireless communications according to one or more standards and/or via one or more transport mediums.

The communication over the network **130** may be performed in accordance with various communication protocols such as Transmission Control Protocol and Internet Protocol (TCP/IP), User Datagram Protocol (UDP), and IEEE communication protocols. In one example, the network **130** may include wireless communications according to Bluetooth specification sets or another standard or proprietary wireless communication protocol. In another example, the network **130** may also include communications over a cellular network, including, e.g., a GSM (Global System for Mobile Communications), CDMA (Code Division Multiple Access), and EDGE (Enhanced Data for Global Evolution) network.

The system **100** is not confined to the components described herein and may include additional or other components, not shown for brevity, which are to be considered within the scope of the embodiments described herein.

The analytics server **110a** may generate and display an electronic platform configured to use various computer models (including artificial intelligence and/or machine-learning models) to identify and display treatment attributes (e.g., RTTP treatment attributes). The electronic platform may include graphical user interfaces (GUI) displayed on each electronic data source **120,** the end-user devices **140,** and/or the administrator computing device **150.** An example of the electronic platform generated and hosted by the analytics server **110a** may be a web-based application or a website configured to be displayed on different electronic devices, such as mobile devices, tablets, personal computers, and the like. In a non-limiting example, a physician operating the physician device **120b** may access the platform, input patient attributes or characteristics and other data, and further instruct the analytics server **110a** to generate an optimized RTTP. The analytics server **110a** may utilize the methods and systems described herein to generate a treatment attribute and display the results on the end-user devices (e.g., the radiotherapy machine **140d)** or adjust the configuration of one of the end-user devices **140.** The analytics server **110a** may display the treatment attribute on the physician device **120b** itself as well.

As described herein, treatment attributes may be or include any attributes related to treating patients at a radiotherapy clinic and/or using a radiotherapy machine. Treatment attributes may include but are not limited to, different treatment modalities, field geometry settings for external beam radiotherapy, side effect predictions, organ and/or tumor segmentation, machine therapy attributes, dosage administration attributes (e.g., dosage amount), treatment frequency, treatment timing, patient's clinical data, etc. A system implementing the systems and methods described herein may provide calibrated predictions for one or more of any such treatment attributes for clinicians and/or radiotherapy machines to implement to treat patients.

The analytics server **110a** may host a website accessible to users operating any of the electronic devices described herein (e.g., end users), where the content presented via the various webpages may be controlled based upon each particular user's role or viewing permissions. The analytics server **110a** may be any computing device comprising a processor and non-transitory machine-readable storage capable of executing the various tasks and processes described herein. Non-limiting examples of such computing devices may include workstation computers, laptop computers, server computers, and the like. While the system **100** includes a single analytics server **110a,** the analytics server **110a** may include any number of computing devices operating in a distributed computing environment, such as a cloud environment.

The analytics server **110a** may execute software applications configured to display the electronic platform (e.g., host a website), which may generate and serve various web pages for each electronic data source **120** and/or end-user device **140.** Different users may use the website to view and/or interact with the predicted results.

The analytics server **110a** may be configured to require user authentication based upon a set of user authorization credentials (e.g., username, password, biometrics, cryptographic certificate, and the like). The analytics server **110a** may access the system database **110b** configured to store user credentials, which the analytics server **110a** may be configured to reference in order to determine whether a set of entered credentials (purportedly authenticating the user) matches an appropriate set of credentials that identify and authenticate the user.

The analytics server **110a** may also store data associated with each user operating one or more electronic data sources **120** and/or end-user devices **140.** The analytics server **110a** may use the data to weigh interactions while training various AI models accordingly. For instance, the analytics server **110a** may indicate that a user is a medical professional whose inputs may be monitored and used to train the machine-learning or other computer models described herein.

The analytics server **110a** may generate and host webpages based upon a particular user's role within the system **100.** In such implementations, the user's role may be defined by data fields and input fields in user records stored in the system database **110b.** The analytics server **110a** may authenticate the user and may identify the user's role by executing an access directory protocol (e.g., LDAP). The analytics server **110a** may generate webpage content that is customized according to the user's role, which is defined by the user record in the system database **110b.**

The analytics server **110a** may receive RTTP data (e.g., patient and treatment data) from a user or retrieve such data from a data repository, analyze the data, and display the results on the electronic platform. For instance, in a non-limiting example, the analytics server **110a** may query and retrieve medical images from the database **120d** and combine the medical images with RTTP data received from a physician operating the physician device **120b.** The analytics server **110a** may then use various models (stored within the system database **110b)** to analyze the retrieved data. The analytics server **110a** then displays the results (e.g., RTTP including couch and gantry angles) via the electronic platform on the administrator computing device, the electronic physician device **120b,** and/or the end-user devices **140.**

The electronic data sources **120** may represent various electronic data sources that contain, retrieve, and/or input data associated with RTTP (e.g., patient data and treatment data). For instance, the analytics server **110a** may use the clinic computer **120a,** physician device **120b,** server **120c** (associated with a physician and/or clinic), and database **120d** (associated with the physician and/or the clinic) to retrieve/receive RTTP data associated with a particular patient's treatment plan.

End-user devices **140** may be any computing device comprising a processor and a non-transitory machine-readable storage medium capable of performing the various tasks and processes described herein. Non-limiting examples of an end-user device **140** may be a workstation computer, laptop computer, tablet computer, or server computer. In operation, various users may use end-user devices **140** to access the GUI operationally managed by the analytics server **110a.** Specifically, the end-user devices **140** may include clinic computer **140a,** clinic database **140b,** clinic server **140c,** a medical device, such as a CT scan machine, radiotherapy machine (e.g., a linear accelerator or a cobalt machine), and the like **(140d),** and a clinic device **140e.**

The administrator computing device **150** may represent a computing device operated by a system administrator. The administrator computing device **150** may be configured to display data retrieved, treatment attributes generated by the analytics server **110a** (e.g., various analytic metrics and/or field geometry) where the system administrator can monitor various models utilized by the analytics server **110a,** electronic data sources **120,** and/or end-user devices **140;** review feedback; and/or facilitate training or calibration of the neural networks that are maintained by the analytic server **110a.**

In operation, a physician may access an application executing on the physician device **120b** and input RTTP data (e.g., patient information, patient diagnosis, and/or radiation therapy treatment attributes, etc.). The analytics server **110a** may then use a patient identifier to query patient data (e.g., patient anatomy and/or medical images) from the electronic data sources **120.** The analytics server may then identify a clinic associated with the patient (e.g., the clinic performing the treatment) and retrieve the neural network that is associated with the clinic (e.g., the neural network that has been calibrated based on a representative set of patient data of the clinic). The analytics server **110a** may then utilize the systems and methods described herein to generate an optimized/uniform RTTP and display the results onto the physician device **120b,** clinic computer **140a,** and/or the medical device **140d** (e.g., a display screen of the radiotherapy machine).

The analytics server **110a** may be in communication (real-time or near real-time) with the medical device **140d,** such that a server/computer hosting the medical device **140d** can adjust the medical device **140d** based on the treatment attributes generated by the analytics server **110a.** For instance, the radiotherapy machine may adjust the gantry and couch based on angles and other attributes determined by the analytics server **110a.** The analytics server **110a** may transmit instructions to the radiotherapy machines indicating any number or type of treatment attributes (e.g., field geometry settings) to facilitate such adjustments.

The analytics server **110a** may store machine-learning models (e.g., neural networks, random forest, support vector machines, etc.) that are trained to predict treatment attributes to treat patients at radiotherapy clinics. The analytics server **110a** may train the machine-learning models using patient data of patients that are treated at radiotherapy machines **170a-n** of the radiotherapy clinics **160.** For instance, the analytics server **110a** may receive patient data from processors of the radiotherapy clinics **160** and generate one or more sets of labeled training data indicating treatment attributes that were used to treat the patients at the respective radiotherapy clinics **160.** The analytics server **110a** may continue to feed the training data into the machine-learning models until the machine-learning models are accurate to a threshold and store the models in a database of the analytics server **110a.**

The machine-learning models stored in the analytics server **110a** may correspond to individual radiotherapy clinics or are otherwise customized based on a particular cohort of patients. For example, each machine-learning model may be associated with an identifier indicating the radiotherapy clinic and may be specific to the guidelines used in that particular clinic. An operator at a radiotherapy clinic may access an end-user device **140** located at the clinic or access an account associated with the clinic. The operator may provide an input at a user interface that causes the end-user device **140** to transmit a request to access a machine-learning model that is associated with the clinic and/or the radiotherapy machines located within the clinic. The request may include an identifier associated with the machine-learning model and/or the clinic that the analytics server **110a** may use as a key in a look-up table to identify the machine-learning model.

The analytics server **110a** may receive the request and, in some cases, after authenticating the user, identify the machine-learning model from the identifier. The analytics server **110a** may transmit the identified machine-learning model to the end-user device **140** or send an alert indicating the end-user device is authorized to access the model.

Upon receipt or access to the machine-learning model, the end-user device **140** may perform the systems and methods described herein to calibrate, customize, and/or fine-tune the identified machine-learning model. For example, the end-user device **140** may generate a customized training data set that includes data that represents the characteristics of patients who are generally treated at the clinic or by a set of radiotherapy machines. The end-user device **140** may then use the customized training data set in order to train the clinic-specific machine-learning model.

Because training data used to train the model has been customized for that particular clinic, the end-user device **140** may avoid displaying overconfident or inaccurate results or results that would be accepted in other clinics but not that the user-user's desired clinic. For example, the end-user device **140** may avoid displaying results that would be accepted in other clinics but not at the user's desired clinic.

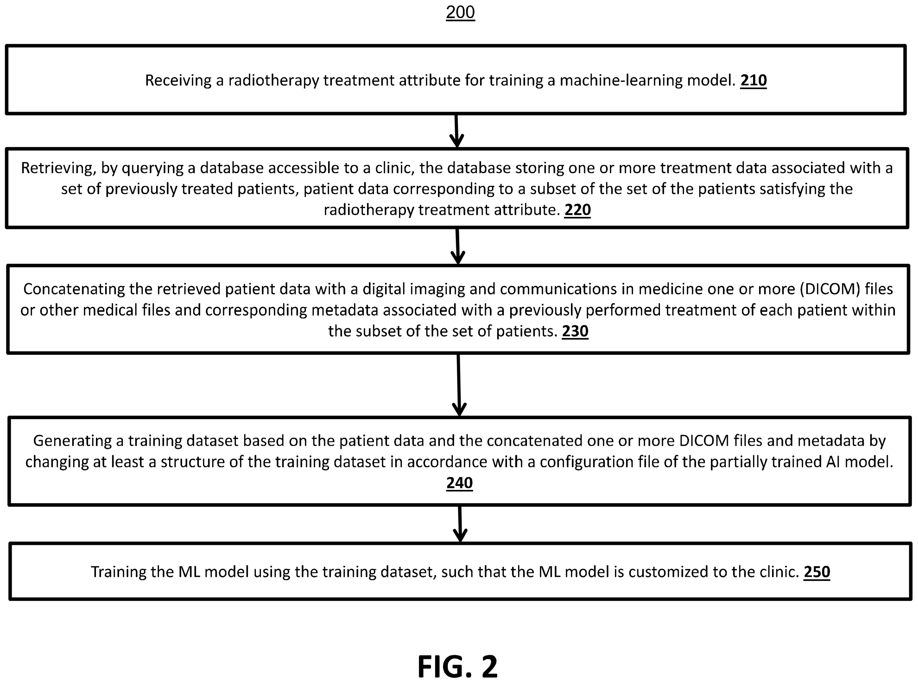

**FIG.** 2 illustrates a flow diagram of a process executed in a clinic-specific radiotherapy planning system, according to an embodiment. The method **200** includes steps **210-250.** However, other embodiments may include additional or alternative steps or may omit one or more steps altogether. The method **200** is described as being executed by a data processing system (e.g., a computer similar to the data source **120,** end-user device **140,** or the analytics server **110a** described in **FIG. 1****).** However, one or more steps of method **200** may be executed by any number of computing devices operating in the distributed computing system described in **FIG. 1****.** For instance, one or more computing devices may locally perform part or all of the steps described in **FIG. 2** or a cloud device may perform such steps.

The method **200** may be used to train a model that is already trained based on a cohort of patient/clinical data and then fine-tune the model to a particular clinic. In some embodiments, the model may only be trained using the data curated via the method **200.**

At step **210,** the analytics server may receive one or more radiotherapy treatment attribute for training the machine-learning model, such that the machine-learning model is fine-tuned or otherwise adapted or customized to the clinic.

In this step, the analytics server may receive a specific attribute or criteria related to the radiotherapy treatment that will guide the training of the machine-learning model. A "radiotherapy treatment attribute" could include various parameters, such as any combination of one or more of: the target location (e.g., head and neck, thorax), prescribed dose levels, organ-at-risk (OAR) constraints, or specific planning techniques (e.g., intensity-modulated radiation therapy, volumetric-modulated arc therapy). These attributes may define what kind of patient data will be used to train the machine-learning model, ensuring that the machine-learning model is fine-tuned to reflect specific clinical practices or treatment protocols. The analytics server may retrieve the attribute from a user input or a pre-configured system setting, such as a configuration file specifying the desired or selected treatment characteristics for the AI model training process.

The treatment attribute could also include more complex considerations, such as specific trade-offs that are characteristic of a clinic's practice, like prioritizing OAR sparing over target dose homogeneity or, conversely, aiming for a highly homogeneous dose distribution within the target at the expense of nearby OARs. The treatment attribute may comprise a trade-off between OAR sparing and target dose homogeneity. In some cases, attributes might be tied to, or be related to, the patient population, such as specific age groups or comorbidities that influence treatment planning. For example, pediatric cancer cases might require tighter constraints to protect developing tissues. The processor's role is to receive and interpret these attributes, which will later be used to filter and select the appropriate patient data for AI. In some embodiments, the analytics server may use a clustering algorithm to generate consistent datasets and remove other datapoints that do not fit in these classes.

At step **220,** the analytics server may retrieve, by querying a clinical database accessible to a clinic, the database storing treatment data associated with a set of previously treated patients, patient data corresponding to a subset of the set of patients satisfying the one or more radiotherapy treatment attribute. The analytics server may retrieve, by querying a clinical database accessible to a clinic, patient data corresponding to a subset of the set of patients satisfying the one or more radiotherapy treatment attribute(s). (The database may store treatment data that is associated with a set of previously treated patients.)

Once the treatment attribute has been received, the analytics server may query a clinical database (a database accessible to the clinic) to retrieve patient data that matches the specified attribute (received at step **210).** The clinical database discussed herein may be a large repository of structured and unstructured data that contains any combination of one or more of: treatment histories, medical imaging, treatment plans, dose distributions, and patient-specific metadata associated with a clinic. In some embodiments, the clinical database may be any data repository accessible to one or more servers of a clinic. In this way, the clinic can use its own data to train the machine learning model.

The analytics server may extract relevant patient data from this vast pool of data included within the clinical database, ensuring the selected records are aligned with the treatment attribute provided in the step **210.** For example, if the machine-learning model is to be trained on thoracic cancer treatments, the analytics server will filter the database to retrieve records for patients treated for lung cancer, esophageal cancer, or other cancers located in the thorax. This query could be highly specific, taking into account not only the anatomical site but also the type of treatment administered (e.g., IMRT, SBRT) and OARs at risk.

The retrieval process may involve complex relational queries that sift through several layers of data, such as identifying relevant treatment plans, patient outcomes, imaging data (CT, MRI, PET scans), and dose information. For instance, if the attribute calls for head and neck cancer cases treated with a specific dose range for the parotid glands, the system may need to extract both the treatment plans and the associated dose-volume histograms (DVHs) for those specific structures. Additionally, the analytics server might exclude certain patient data that doesn't meet the treatment attribute, such as patients who were treated with a different, e.g. an entirely different, modality (e.g., proton therapy instead of photon therapy) or patients with incomplete treatment records. This ensures that the dataset used for AI model training is both relevant and comprehensive.

In some embodiments, the analytics server may, after extracting and mapping the digital imaging and communications in medicine DICOM files of the patients (or other medical files), classify the data into different subsets of the data that are consistent. For instance, the analytics server may use a clustering algorithm and divide the data into different subsets where each subset includes a relatively similar group of patients (e.g., patients that have a common attribute that satisfies a similarity threshold). In some embodiments, the data used for clustering may be first extracted or computed from the files (e.g., the DVH curves computed from the DICOM dose file using the contours from the structure DICOM file).

At step **230,** the analytics server may concatenate the retrieved patient data with one or more DICOM files or other medical files and corresponding metadata associated with a previously performed treatment of each patient within the subset of the set of patients.

Though aspects of the embodiments discussed herein are described in terms of DICOM files, the methods and system discussed herein can be implemented using any medical file or other files that include patient data (e.g., any medical file). Therefore, the methods and systems discussed herein are not limited to DICOM files.

After retrieving the relevant patient data, the analytics server may proceed to combine, or "concatenate," this data with the corresponding Digital Imaging and Communications in Medicine (DICOM) files and related metadata. DICOM files may store various data associated with radiotherapy treatments, such as any combination of one or more of: medical images (e.g., CT, MRI, and the like), treatment plans, dose distributions, and organ contouring information. By concatenating the data with the corresponding DICOM files (or data included within the DICOM files), the analytics server may allow for the machine-learning model to be trained using both clinical records and the medical imaging data necessary for treatment planning. For example, if a retrieved patient was treated for lung cancer, the corresponding DICOM files may include their CT scans, the contoured lung tumor, and nearby OARs, and the detailed radiation dose plan used during treatment. As discussed herein, the machine-learning model may be trained using both of these features, which will allow for an improved machine-learning model.

The concatenation of patient data with DICOM files and metadata may provide operational efficiencies by allowing the machine-learning model to have access to all aspects of the treatment process while training. As used herein, metadata associated with the DICOM files may include any combination of one or more of: key patient identifiers, imaging parameters, treatment dates, and organ-at-risk delineations. By combining these elements, the analytics server may create a more comprehensive training dataset for the machine-learning model, allowing the machine-learning model to learn from both the clinical decision-making process and the associated imaging data.

In some embodiments, the analytics server may execute a data normalization or organization protocol to ensure the files are in the appropriate format for training. For example, the analytics server may organize the DICOM files into a data structured folder hierarchy that mirrors how the machine-learning model expects to receive its inputs, such as separating dose distributions from structure sets.

In some embodiments, the analytics server may execute one or more deduplication protocol after determining that a particular patient's treatment is included in the training dataset more than once. For instance, the analytics server may compare patient data and determine that two datasets satisfy a similarity threshold. As a result, the analytics server may remove one of the similar datasets. In some embodiments, the analytics server may analyze the patient data and determine one or more outliers (data points that are different than other data points within the training dataset more than a certain threshold). As a result, the analytics server may remove the outlier data point.

At step **240,** the analytics server may generate a training dataset based on the patient data, and the one or more concatenated DICOM files and metadata, by changing at least a file structure of the training dataset in accordance with a configuration file of the pre-trained trained AI model.

Once the patient data, DICOM files, and metadata are combined, the analytics server may generate a structured training dataset for the machine-learning model to ingest. This process may involve reformatting or restructuring the data to meet the specific input requirements of the machine-learning model, e.g., as outlined in its configuration file. For instance, the machine-learning model might require patient data to be organized by treatment phases, such as separating initial treatments from retreatment cases or distinguishing between simultaneous integrated boost plans and sequential boost plans for the cases where multiple targets need to be treated. The analytics server may also cluster the data according to various treatment attributes, ensuring that the training dataset is tailored to the specific learning goals of the AI model.

The data restructuring discussed herein may allow for the machine-learning model to properly interpret the data and generate accurate predictions during training. For example, the system might standardize the representation of OAR volumes, convert dose metrics into normalized values, or filter out any irrelevant or noisy data, such as incomplete treatment records or extreme outliers. In some embodiments, the analytics server may apply specific algorithms to group similar cases (and their corresponding data), ensuring that the dataset remains homogeneous beyond a defined threshold. Generating a homogeneous training dataset may improve the machine-learning model's ability to be trained efficiently.

At step **250,** the analytics server may continue to train the pre-trained AI model using the training dataset. After generating the structured training dataset, the analytics server may train the machine-learning model using the newly prepared data. The machine-learning model may already have been pre-trained on general datasets or historical clinic data. However, the analytics server may allow the machine-learning model to be fine-tuned to reflect the specific clinical practices of the current clinic or hospital. The training process may involve supervised learning techniques, where the usage of the machine-learning model may lead to optimal radiation treatment plans based on the newly structured dataset.

As the machine-learning model processes the training data, it may adjust its internal parameters to improve its predictions and treatment planning recommendations. This training may involve multiple iterations, where the machine-learning model's predictions are compared against actual clinical outcomes or certain features from previous treatments, such as DVH curves. The machine-learning model may become better equipped to generate treatment plans that align with the clinic's specific protocols, preferences, and/or trade-offs between tumor control and OAR sparing.

Using the method **200,** a clinic can receive a machine learning model from a central entity and fine-tune or otherwise adapt the trained model to their own clinic using the clinic's specially segmented data.

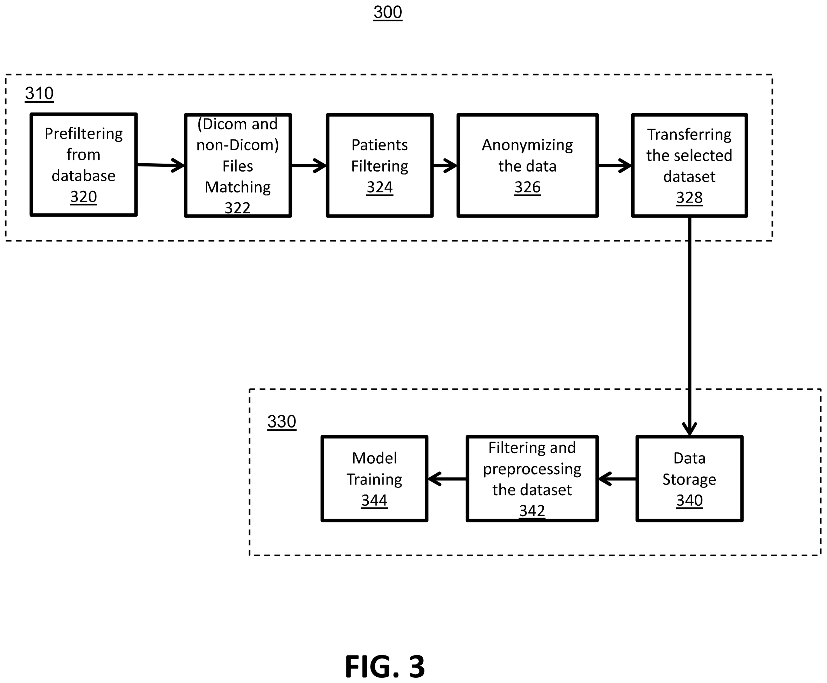

Referring now to **FIG. 3****,** a non-limiting example **300** for customizing a machine-learning model based on a particular clinic's data is illustrated. In the example **300,** a user (e.g., a medical professional) may implement the methods and systems discussed herein to customize a machine-learning model to a particular clinic to predict 3D dose distributions (or other treatment attributes) for patients. The user may use the example **300** to generate a training dataset that will allow the machine-learning model to be customized for the particular clinic. In this non-limiting example, the user is customizing the model based on lung cancer patients.

The user may generate the training data in the step **310** and then train the model in the step **330.** The user may use a platform of the clinic where a processor/server associated with the clinic is performing the steps discussed in **FIG. 3****.** In the step **310,** the processor may first employ various automated scripts, such as SQL or Python, to prefilter patient data within the clinic's database (step **320).** The processor may prefilter the data based on the inputs (e.g., keywords) provided by the user. The focus here may be to select patients who have undergone treatment for lung cancer (at the clinic), where precise dose distribution is critical. The processor may generate a reference table comprising patient metadata specific to lung cancer treatments, streamlining the selection process and ensuring that only relevant data is processed in subsequent steps.

Following the prefiltering step **320,** the processor may retrieve the corresponding DICOM and non-DICOM files that match the prefiltered patients. These files may include CT images, radiation plans, and/or dose files stored in the clinic's DICOM archive as well as other types of data such as genomics, proteomics. The processor may use term matching or other identification protocols to match the DICOM and non-DICOM files for the patients that have been prefiltered. This step ensures that the data collection is both accurate and limited to the necessary patient information, thus optimizing the data management process.

At step **324,** the processor may display a list of the patients prefiltered to the user, where the user can further select a subset (or sometimes all) of the patients.

At the optional step **326,** the processor may use various protocols to anonymize the data associated with the patients selected in the step **324.** In some embodiments, the step **326** may be performed if the data is intended for use outside the clinic, such as in collaborative research projects or external data analysis. In this case, standard anonymization tools may be utilized to remove personally identifiable information from both DICOM and metadata files, ensuring compliance with privacy regulations and ethical standards.

Once the relevant and optionally anonymized data is compiled, the processor may aggregate the data and generate a training dataset based on the compiled and aggregated data. The processor may then transmit the training dataset to a dedicated data storage **340** in the step **328.** The data storage may be hosted on a cloud platform or a specialized local server, depending on the clinic's infrastructure. The transfer may be facilitated through automated protocols that not only secure the data but also maintain its integrity during the transfer process.

At the step **342,** the processor may structure and organize the data into a specific structure required by the machine-learning model. This process may involve selecting a single CT series per patient and aligning it with the corresponding plan and dose information. Additional tools for data analysis, such as outlier detection and/or clustering into consistent subsets, may be employed to refine the data, e.g. to refine the training dataset, further. This ensures that the dataset is optimally prepared for effective model training. With the data fully prepared, the clinic's data scientists or an automated system can transfer the data to the machine-learning model training procedure (step **344).**

The various illustrative logical blocks, modules, circuits, and algorithm steps described in connection with the embodiments disclosed herein may be implemented as electronic hardware, computer software, or combinations of both. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. Skilled artisans may implement the described functionality in varying ways for each particular application, but such implementation decisions should not be interpreted as causing a departure from the scope of this disclosure or the claims.

Embodiments implemented in computer software may be implemented in software, firmware, middleware, microcode, hardware description languages, or any combination thereof. A code segment or machine-executable instructions may represent a procedure, a function, a subprogram, a program, a routine, a subroutine, a module, a software package, a class, or any combination of instructions, data structures, or program statements. A code segment may be coupled to another code segment or a hardware circuit by passing and/or receiving information, data, arguments, parameters, or memory contents. Information, arguments, parameters, data, etc. may be passed, forwarded, or transmitted via any suitable means including memory sharing, message passing, token passing, network transmission, etc.

The actual software code or specialized control hardware used to implement these systems and methods is not limiting of the claimed features or this disclosure. Thus, the operation and behavior of the systems and methods were described without reference to the specific software code being understood that software and control hardware can be designed to implement the systems and methods based on the description herein.

When implemented in software, the functions may be stored as one or more instructions or code on a non-transitory computer-readable or processor-readable storage medium. The steps of a method or algorithm disclosed herein may be embodied in a processor-executable software module, which may reside on a computer-readable or processor-readable storage medium. A non-transitory computer-readable or processor-readable media includes both computer storage media and tangible storage media that facilitate transfer of a computer program from one place to another. A non-transitory processor-readable storage media may be any available media that may be accessed by a computer. By way of example, and not limitation, such non-transitory processor-readable media may comprise RAM, ROM, EEPROM, CD-ROM or other optical disk storage, magnetic disk storage or other magnetic storage devices, or any other tangible storage medium that may be used to store desired program code in the form of instructions or data structures and that may be accessed by a computer or processor. Disk and disc, as used herein, include compact disc (CD), laser disc, optical disc, digital versatile disc (DVD), floppy disk, and Blu-ray disc where disks usually reproduce data magnetically, while discs reproduce data optically with lasers. Combinations of the above should also be included within the scope of computer-readable media. Additionally, the operations of a method or algorithm may reside as one or any combination or set of codes and/or instructions on a non-transitory processor-readable medium and/or computer-readable medium, which may be incorporated into a computer program product.

The preceding description of the disclosed embodiments is provided to enable any person skilled in the art to make or use the embodiments described herein and variations thereof. Various modifications to these embodiments will be readily apparent to those skilled in the art, and the principles defined herein may be applied to other embodiments without departing from the scope of the subject matter disclosed herein. Thus, the present disclosure is not intended to be limited to the embodiments shown herein but is to be accorded the widest scope consistent with the following claims and the principles and novel features disclosed herein.

While various aspects and embodiments have been disclosed, other aspects and embodiments are contemplated. The various aspects and embodiments disclosed are for purposes of illustration and are not intended to be limiting, with the true scope being indicated by the following claims.

## Claims

1. A method for training and integrating a machine learning, ML, model for radiation therapy treatment planning, the method comprising:
receiving, by at least one processor, one or more radiotherapy treatment attribute for training the ML model;
retrieving, by the at least one processor querying a database accessible to a clinic, the database storing treatment data associated with a set of previously treated patients, patient data corresponding to a subset of the set of the patients satisfying the one or more radiotherapy treatment attribute;
concatenating, by the at least one processor, the retrieved patient data with one or more digital imaging and communications in medicine files or other medical files and corresponding metadata associated with a previously performed treatment of each patient within the subset of the set of patients;
generating, by the at least one processor, a training dataset based on the patient data and the concatenated one or more digital imaging and communications in medicine files or other medical files and metadata by changing at least a file structure of the training dataset in accordance with a configuration file of the ML model; and
training, by the at least one processor, the ML model using the training dataset, such that the ML model is customized to the clinic.

2. The method of claim 1, wherein the machine learning model is only trained using the training dataset.

3. The method of claim 1 or claim 2, wherein the machine learning model was previously trained using a secondary training dataset and is fine-tuned for the clinic.

4. The method of any preceding claim, further comprising:
anonymizing, by the at least one processor, at least one of the patient data, the concatenated digital imaging and communications in medicine files or other medical files, or the metadata.

5. The method of any preceding claim, further comprising:
clustering, by the at least one processor, the training dataset into a plurality of consistent subsets;
and/or:
the method further comprising:
when an outlier data point is identified, removing, by the at least one processor, the outlier data point within the training dataset.

6. The method of any preceding claim, wherein the radiotherapy treatment attribute corresponds to a specific treatment technique;
and/or:
the method further comprising:
de-duplicating, by the at least one processor, the training dataset by removing data associated with patients that satisfy a similarity threshold.

7. A computer-readable medium for training and integrating a machine learning, ML, model for radiation therapy treatment planning, the computer-readable medium comprising instructions that when executed cause a processor to:
receive one or more radiotherapy treatment attribute for training the ML model;
retrieve by the at least one processor querying a database accessible to a clinic, the database storing treatment data associated with a set of previously treated patients, patient data corresponding to a subset of the set of the patients satisfying the one or more radiotherapy treatment attribute;
concatenate the retrieved patient data with one or more digital imaging and communications in medicine files or other medical files and corresponding metadata associated with a previously performed treatment of each patient within the subset of the set of patients;
generate a training dataset based on the patient data and the concatenated one or more digital imaging and communications in medicine files or other medical files and metadata by changing at least a file structure of the training dataset in accordance with a configuration file of the ML model; and
train the ML model using the training dataset, such that the ML model is customized to the clinic.

8. The computer-readable medium of claim 7, wherein the machine learning model is only trained using the training dataset.

9. The computer-readable medium of claim 7 or claim 8, wherein the machine learning model was previously trained using a secondary training dataset and is fine-tuned for the clinic.

10. The computer-readable medium of any of claim 7 to claim 9, wherein the instructions further cause the processor to anonymize at least one of the patient data, the concatenated digital imaging and communications in medicine files or other medical files, or the metadata.

11. The computer-readable medium of any of claim 7 to claim 10, wherein the instructions further cause the processor to cluster the training dataset into a plurality of homogeneous subsets;
and/or:
wherein the instructions further cause the processor to, when an outlier data point is identified, remove the outlier data point within the training dataset.

12. The computer-readable medium of any of claim 7 to claim 11, wherein the radiotherapy treatment attribute corresponds to a specific treatment technique;
and/or:
wherein the instructions further cause the processor to de-duplicate the training dataset by removing data associated with patients that satisfy a similarity threshold.

13. A computer system for training and integrating a machine learning, ML, model for radiation therapy treatment planning, the computer system comprising a processor configured to:
receive one or more radiotherapy treatment attribute for training the ML model;
retrieve by the at least one processor querying a database accessible to a clinic, the database storing treatment data associated with a set of previously treated patients, patient data corresponding to a subset of the set of the patients satisfying the one or more radiotherapy treatment attribute;
concatenate the retrieved patient data with one or more digital imaging and communications in medicine files or other medical files and corresponding metadata associated with a previously performed treatment of each patient within the subset of the set of patients;
generate a training dataset based on the patient data and the concatenated one or more digital imaging and communications in medicine files or other medical files and metadata by changing at least a file structure of the training dataset in accordance with a configuration file of the ML model; and
train the ML model using the training dataset, such that the ML model is customized to the clinic.

14. The computer system of claim 13, wherein the machine learning model is only trained using the training dataset.

15. The computer system of claim 13 or claim 14, wherein the machine learning model was previously trained using a secondary training dataset and is fine-tuned for the clinic;
and/or:
wherein the processor is further configured to anonymize at least one of the patient data, the concatenated digital imaging and communications in medicine files or other medical files, or the metadata.
